(19) Europäisches Patentamt
European Patent Office
Office européen des brevets

(11) Publication number: 0 132 869
A1

(12) # EUROPEAN PATENT APPLICATION

(21) Application number: 84200927.6

(22) Date of filing: 26.06.84

(51) Int. Cl.[4]: C 08 F 2/50
G 03 F 7/10

(30) Priority: 27.06.83 US 507885

(43) Date of publication of application:
13.02.85 Bulletin 85/7

(84) Designated Contracting States:
AT BE CH DE FR GB IT LI NL SE

(71) Applicant: STAUFFER CHEMICAL COMPANY

Westport Connecticut 06880(US)

(72) Inventor: Via, Francis Anthony
2389 Ridge Street
Yorktown Heights New York 10598(US)

(74) Representative: Urbanus, Henricus Maria, Ir. et al,
c/o Vereenigde Octrooibureaux Nieuwe Parklaan 107
NL-2587 BP 's-Gravenhage(NL)

(54) Alpha-halogenated aromatic compounds as photoinitiators.

(57) Alpha-halogenated aromatic compounds of the formula

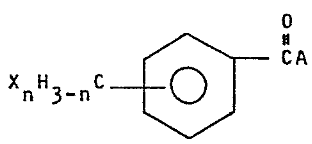

where A is selected from lower alkyl, alkoxy and hydroxy, X being halogen and n being 1 to 3 have been found to be photointiators for use in the photopolymerization of monomeric and polymeric compositions of photopolymerizable substances.

EP 0 132 869 A1

Croydon Printing Company Ltd

# ALPHA-HALOGENATED AROMATIC COMPOUNDS AS PHOTOINITIATORS

## Field of the Invention

The present invention relates to processes and compositions for photopolymerization using certain alpha-halogenated aromatic compounds as photoinitiators.

## Background of the Invention

Photopolymerization of unsaturated compositions in which a photoinitiating compound is included in the polymerizable mass is well known in the art. The process has many advantages over thermal polymerization and is particularly useful where long shelf life combined with rapid hardening at low temperature is desirable. Photoinitiating compounds must absorb light and utilize the energy so acquired to initiate polymerization.

A large number of compounds have been found useful as photoinitiators for the polymerization of unsaturated compounds. Among those theretofore in most common usage in industry are the benzoin ethers of primary and secondary alcohols such as methyl alcohol, ethyl alcohol, isopropyl alcohol and isobutyl alcohol. Also, compounds such as phenyl glyoxal and 1-phenyl butane-1, 2-dione are disclosed as photosensitizers in U.S. Patent No. 2,413,973. Additionally, various acetophenone compounds such as 2,2-diethoxyacetophenone are claimed to have photoinitiating capability in U.S. Patent No. 3,715,293.

In U.S. Patent No. 3,933,682 photoinitiators containing an aromatic ring with carbonyl group substitution are described in admixture with at least one

organic compound of a Group V element and at least one halogenated hydrocarbon. The patent indicates at Col. 2, line 13 that halogenated aceto- and benzophenone compounds can be used without further defining their structure. U.S. Patent No. 4,188,455 describes compounds containing an unsubstituted aromatic ring with a carbonyl group attached thereto which contains at least one -C-OH or -C-alkoxy substituent. U.S. Patent No. 4,229,274 mentions various acetophenone derivatives including trichloroacetophenone compounds. U.S. Patent No. 4,239,609 describes an activated photoinitiator system containing at least one aromatic carbonyl compound and an dichloromethyl aromatic activator which is not disclosed as having photoinitiator utility.

<u>Summary of the Present Invention</u>

It has now been found that certain alpha-halogenated aromatic compounds are effective as photoinitiators in monomeric and polymeric compositions of photopolymerizable substances. The photoinitiators used in connection with the present invention have the following formula:

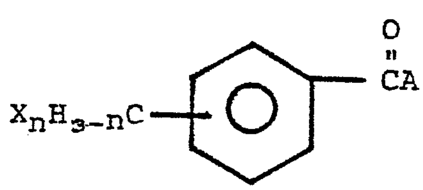

where A is selected from the group consisting of lower alkyl (e.g., $C_1$-$C_6$ alkyl), alkoxy and hydroxy and X is halogen with n being an integer of from 1 to 3, preferably 1 or 2.

Detailed Description of the Present Invention

The compositions curable by actinic radiation according to the invention can contain a photopolymerizable polymer in a reactive ethylenically unsaturated monomeric medium, a reactive polymer alone, a reactive monomer alone, or any of these combined with an inert solvent. Additionally, the polymerizable composition can contain any of the pigments commonly used in photopolymerization techniques.

The process can be carried out by mixing a quantity of photoinitiating compound of the present invention with a photopolymerizable composition and exposing the resultant mixture to actinic radiation. Alternatively, a one-component system comprising the photopolymerizable composition, the photoinitiator of the invention and, if desired, pigmentation, can be formed.

The photoinitiating compounds of the invention are suitable in the actinic light curing of unsaturated monomeric compounds either alone or as copolymerizable constituents of unsaturated polymer/monomer systems. Such systems are composed of mixtures of conventional unsaturated polymers and unsaturated monomers.

Monomers which are useful in practicing the invention are acrylic, α-alkacrylic and α-chloroacrylic acid compounds such as esters, amides and nitriles. Examples of such compounds are acrylonitrile, methacrylonitrile, methyl acrylate, ethyl acrylate, methyl methacrylate, isobutyl methacrylate, 2-ethylhexyl acrylate, methacrylamide and methyl α-chloroacrylate. Also useful, although not preferred due to their slower rates of reactivity, are vinyl and vinylidene esters, ethers and ketones. Additionally, compounds having more than

one terminal unsaturation can be used. Examples of these include diallyl phthalate, diallyl maleate, diallyl fumarate, triallyl cyanurate, triallyl phosphate, ethylene glycol dimethacrylate, glycerol trimethacrylate, pentaerythritol triacrylate, pentaerythritol tetraacrylate, trimethylolpropane triacrylate, methacrylic anhydride, and allyl ethers of monohydroxy or polyhydroxy compounds, such as ethylene glycol diallyl ether, pentaerythritol tetraallyl ether, and the like. Non-terminally unsaturated compounds such as diethyl fumarate can similarly be used.

The acrylic acid derivatives are particularly well suited to the practice of the invention and are consequently preferred components as monomers in monomer-containing polymerizable systems and as reactive centers in polymerizable polymers.

A preferred manner of practicing the invention is by the use of photopolymerizable molding and coating compositions which comprise mixtures of unsaturated polymeric compounds and monomeric compounds copolymerizable therewith. The polymeric compounds can be conventional polyesters prepared from unsaturated polycarboxylic acids such as maleic acid, fumaric acid, glutaconic acid, itaconic acid, citraconic acid, mesaconic acid, and the like, and polyhydric alcohols such as ethylene glycol, diethylene glycol, glycerol, propylene glycol, 1,2-butanediol, 1,4-butanediol, pentaerythritol, trimethylolpropane and the like. The carboxylic acid content can also contain saturated components. The

inclusion of a monobasic fatty acid content, either as such or in the form of a triglyceride or oil, in the photopolymerizable polyester composition to comprise an alkyd resin is also acceptable. These resins can, in turn, be modified by silicones, epoxides, isocyanates, etc., by known techniques.

Additionally, the photopolymerizable composition can contain a sensitizer capable of enhancing the photoinitiating reactivity of the photoinitiating compound of the invention. Examples of sensitizers useful in the practice of the invention are such compounds as xanthone, thioxanthone, acetophenone and the like. These are typically added in amounts ranging from about 0.1 to about 6 weight percent. The techniques whereby such sensitizers are selected for use in conjunction with particular photoinitiators are well known in the art. See for example, Murov, Handbook of Photochemistry, Marcel Dekker, Inc., New York (1973).

Thus it is seen that the constitution of photopolymerizable compositions which can be used in the practice of the invention is widely variable. However, the compounds enumerated above are purely illustrative. Materials subject to polymerization by actinic radiation as well as permissible variations and substitutions of equivalent components within particular types of compositions are well known to those skilled in the art.

The compounds which function as photoinitiators in accordance with the present invention are known for other uses and are easily synthesized by known means.

The photoinitiators of the invention can be utilized in amounts ranging from 0.01 to about 30% by weight based on the photopolymerizable composition. However, preferable amounts of the compounds are from about 0.5

to about 20 weight percent with optimal results being achieved with amounts in the range of from about 1.0 to about 16 weight percent.

An acceptable source of actinic light radiation is any apparatus which emits light radiation in the approximate region of from about 2000 Angstroms to about 8000 Angstroms and preferably from about 2400 Angstroms to about 5400 Angstroms. One such apparatus is PPG Model QC 1202, a UV processor, manufactured by PPG Industries, Inc.

The present invention is illustrated by the Example which follows.

TEST PROCEDURE USED IN THE EXAMPLES

The induced rate of photopolymerization of a standard test solution of acrylate/alkyd resin was measured for each of the compounds which was tested. This standard test solution consisted of 42%, by weight, trimethylolpropane triacrylate (TMPTA), 17%, by weight, of ethylhexyl acrylate (EHA), and 41%, by weight, of an unsaturated long chain linseed oil alkyl resin (ACTOMER X-80 brand).

The test solution was exposed to light radiation from a light source (PPG Model QC 1202) which contained two high intensity, medium pressure quartz mercury lamps, 12 inches in length, with each operating at a linear power density of about 200 watts per inch or 2400 watts per lamp. The lamps were housed in an elliptical reflector above a variable speed conveyor belt so that each lamp provided a 2-inch band of high flux actinic radiation on the conveyor. This 2-inch exposure area was bordered on both sides by an additional 2-inch area of medium flux energy for a total radiation area of 6 inches for each lamp. In the curing data presented herein, the cure rate of the polymerizable compositions is presented in feet-per-minute-per-lamp (ft./min./lamp). Thus a conveyor belt speed of one foot/min. will, with a 12-inch exposure area for the two lamps, provide 60 seconds of exposure or a cure rate of 0.5 ft./min./lamp. Similarly, a belt speed of 10 ft./min./lamp, while a speed of 20.0 ft./min. will give 3 seconds exposure or a rate of 10 ft./min./lamp.

The extent of curing was determined by a standard pencil hardness test with all samples coated on aluminum plate to a thickness of 2 mils and polymerized to achieve a standard pencil hardness of from 4H to 6H where this was attainable.

EXAMPLES 1-2

These Examples illustrate the photoefficienty of various alpha-chlorinated aromatic compounds when used at 4 wt. % loading in the standard test solution:

| Compound | Cure Rate (feet/min./lamp) |
|---|---|
| p-(dichloromethyl)acetophenone | 8 |
| p-(chloromethyl)benzoic acid | 3 |

The foregoing Examples are intended to merely be illustrative of certain embodiments of the present invention and should not be construed in a limiting manner. The scope of protection that is sought is set forth in the claims which follow.

What is Claimed:

1. In the photopolymerization of monomeric and polymeric compositions of photopolymerizable substances wherein a photoinitiator is admixed with a photopolymerizable composition and the mixture exposed to actinic radiation, the improvement wherein photopolymerization is effectively initiated by a compound of the formula:

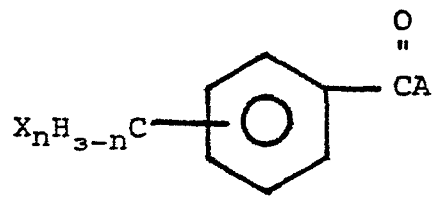

where A is selected from the group consisting of lower alkyl, alkoxy and hydroxy and X is halogen with n being 1-3.

2. The method of Claim 1 where the compound is p-(dichloromethyl)acetophenone.

3. The method of Claim 1 where the compound is p-(chloromethyl)benzoic acid.

4. The method of Claim 1 wherein the compound is present at a concentration of about 0.5 percent by weight to about 20 percent by weight.

5. The method of Claim 1 wherein the compound is present at a concentration of about 1.0 percent by weight to about 16 percent by weight.

6. The method of Claim 2 wherein the compound is present at a concentration of about 0.5 percent by weight to about 20 percent by weight.

7. The method of Claim 2 wherein the compound is present at a concentration of about 1.0 percent by weight to about 16 percent by weight.

8. The method of Claim 3 wherein the compound is present at a concentration of about 0.5 percent by weight to about 20 percent by weight.

9. The method of Claim 3 wherein the photoinitiating compound is present at a concentration of about 1.0 percent by weight to about 16 percent by weight.

10. A composition photopolymerizable by actinic radiation comprising unsaturated polymerizable consituents containing dispersed there in an effective amount of a photoinitiating compound of the formula:

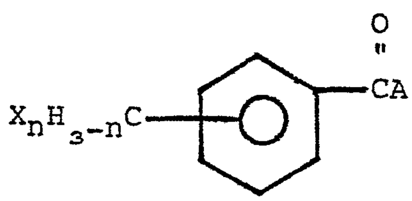

where A is selected from the group consisting of lower alkyl, alkoxy and hydroxy and X is halogen with n being 1 to 3.

11. The composition of Claim 10 where the compound is p-(dichloromethyl)acetophenone.

12. The composition of Claim 10 where the compound is p-(chloromethyl)benzoic acid.

13. The composition of Claim 10 wherein the compound is present at a concentration of about 0.5 percent by weight to about 20 percent by weight.

14. The composition of Claim 10 wherein the compound is present at a concentration of about 1.0 percent by weight to about 16 percent by weight.

15. The composition of Claim 11 wherein the compound is present at a concentration of about 0.5 percent by weight to about 20 percent by weight.

16. The composition of Claim 11 wherein the photoinitiating compound is present at a concentration of about 1.0 percent by weight to about 16 percent by weight.

17. The composition of Claim 12 wherein the compound is present at a concentration of about 0.5 percent by weight to about 20 percent by weight.

18. The composition of Claim 12 wherein the photoinitiating compound is present at a concentration of about 1.0 percent by weight to about 16 percent by weight.

European Patent Office

# EUROPEAN SEARCH REPORT

0132869

Application number

EP 84 20 0927

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-3 374 160 (TZU JEN MAO)<br>---- | | C 08 F 2/50<br>G 03 F 7/10 |
| A | US-A-3 251 759 (H.R. ANDERSON)<br>----- | | |
| | | | TECHNICAL FIELDS SEARCHED (Int. Cl. 3) |
| | | | C 08 F<br>G 03 F |

The present search report has been drawn up for all claims

| Place of search | Date of completion of the search | Examiner |
|---|---|---|
| THE HAGUE | 17-10-1984 | CAUWENBERG C.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document